(19) Europäisches Patentamt
European Patent Office
Office européen des brevets

(11) Veröffentlichungsnummer: **0 046 267**
**A1**

# (12) EUROPÄISCHE PATENTANMELDUNG

(21) Anmeldenummer: **81106257.9**

(22) Anmeldetag: **11.08.81**

(51) Int. Cl.³: **C 07 D 487/04**, C 07 D 491/14, A 61 K 31/505 // C07C87/28, C07D239/84, (C07D487/04, 239/00, 235/00)

(30) Priorität: **15.08.80 CH 6192/80**
**27.05.81 CH 3483/81**

(43) Veröffentlichungstag der Anmeldung: **24.02.82**
**Patentblatt 82/8**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Chodnekar, Madhukar Subraya, Dr., Rebhaldenstrasse 6, CH-4411 Seltisberg (CH)**
Erfinder: **Kaiser, Ado, Dr., Hirsweg 3, CH-4415 Lausen (CH)**
Erfinder: **Kienzle, Frank, Dr., Tannwaldweg 9, CH-4113 Flüh (CH)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

(54) **Neue Imidazochinazolinderivate, ihre Herstellung und Verwendung, und Arzneimittel enthaltend diese Derivate.**

(57) Es werden neue Imidazochinazoline der Formel

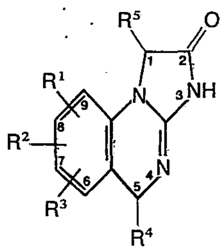

I

worin $R^1$, $R^2$ und $R^3$ Wasserstoff, Halogen, $C_{1-4}$-Alkyl, $C_{1-4}$-Alkoxy oder $C_{2-5}$-Alkoxyalkyl, oder zwei benachbarte Reste $R^1$, $R^2$ oder $R^3$ Methylendioxy oder Äthylendioxy, und $R^4$ und $R^5$ Wasserstoff oder $C_{1-4}$-Alkyl sind, und deren Tautomere, sowie physiologisch verträgliche Säureadditionssalze solcher Verbindungen hergestellt.

Diese Verbindungen hemmen die Aggregation der Blutplättchen und/oder die Magensäuresekretion und/oder sind kreislaufwirksam.

EP 0 046 267 A1

0046267

F.Hoffmann-La Roche & Co. Aktiengesellschaft,Basel,Schweiz

11. Aug. 1981

RAN 4044/56

# Neue Imidazochinazolinderivate, ihre Herstellung und Verwendung, und Arzneimittel enthaltend diese Derivate.

Die vorliegende Erfindung betrifft neue Imidazochinazoline der Formel

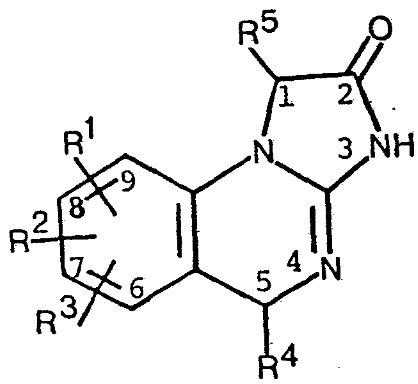

I

worin $R^1$, $R^2$ und $R^3$ Wasserstoff, Halogen, $C_{1-4}$-Alkyl, $C_{1-4}$-Alkoxy oder $C_{2-5}$-Alkoxyalkyl; oder zwei benachbarte Reste $R^1$, $R^2$ oder $R^3$ Methylendioxy oder Aethylendioxy; und $R^4$ und $R^5$ Wasserstoff oder $C_{1-4}$-Alkyl sind, und deren Tautomere, sowie physiologisch verträgliche Säureadditionssalze solcher Verbindungen.

Der hier verwendete Ausdruck $C_{1-4}$-Alkyl bzw. $C_{1-4}$-Alkoxy bezieht sich auf geradkettige oder verzweigte Reste, wie Methyl, Aethyl, Propyl, Isopropyl, Butyl und die ent-

Mé/ 18.6.81

sprechenden Alkoxyreste. Der Ausdruck $C_{2-5}$-Alkoxyalkyl bezieht sich auf geradkettige oder verzweigte Reste, wie Methoxymethyl. Der hier verwendete Ausdruck Halogen bezieht sich auf Chlor, Brom, Fluor und Jod.

Unter den Verbindungen der Formel I sind diejenigen bevorzugt, worin $R^1$ und $R^2$ Wasserstoff, Halogen, $C_{1-4}$-Alkyl oder $C_{1-4}$-Alkoxy in 6- und 7-Stellung, insbesondere diejenigen, worin $R^1$ und $R^2$ Wasserstoff, Chlor, Brom, Methyl oder Methoxy in 6- und 7-Stellung und $R^3$ Wasserstoff sind. Besonders bevorzugt sind Verbindungen der Formel I, worin $R^1$ 6-Chlor, $R^2$ 7-Chlor und $R^3$ Wasserstoff ist.

Bevorzugt sind ferner die Verbindungen der Formel I, worin $R^4$ und $R^5$ Methyl oder Wasserstoff sind.

Beispiele solcher bevorzugten Verbindungen sind:

6,7-Dichlor-4,5-dihydroimidazo[1,2-a]chinazolin-2(1H)-on

6-Chlor-4,5-dihydroimidazo[1,2-a]chinazolin-2(1H)-on, deren Tautomere, sowie Mineralsäuresalze dieser Verbindungen.

Beispiele von Verbindungen der Formel I sind:

7-Brom-6-methyl-4,5-dihydroimidazo[1,2-a]chinazolin-2(1H)-on,

6,7-Dimethyl-4,5-dihydroimidazo[1,2-a]chinazolin-2(1H)-on,

7-Chlor-4,5-dihydroimidazo[1,2-a]chinazolin-2(1H)-on,

7-Methoxy-4,5-dihydroimidazo[1,2-a]chinazolin-2(1H)-on,

7-Chlor-6-methyl-4,5-dihydroimidazo[1,2-a]chinazolin-2(1H)-on,

7-Methyl-4,5-dihydroimidazo[1,2-a]chinazolin-2(1H)-on,

6-Methyl-4,5-dihydroimidazo[1,2-a]chinazolin-2(1H)-on,

4,5-Dihydroimidazo[1,2-a]chinazolin-2(1H)-on,

6,7,8-Trimethoxy-4,5-dihydroimidazo[1,2-a]chinazolin-2(1H)-on,

6-Chlor-7-methoxy-4,5-dihydroimidazo[1,2-a]chinazolin-2(1H)-on,

8-Chlor-4,5-dihydroimidazo[1,2-a]chinazolin-2(1H)-on,

9-Methyl-4,5-dihydroimidazo[1,2-a]chinazolin-2(1H)-on,

6,7-Dimethoxy-4,5-dihydroimidazo[1,2-a]chinazolin-2(1H)-on,

1-Methyl-6-chlor-7-methoxy-4,5-dihydroimidazo[1,2-a]chinazolin-2(1H)-on,

5-Methyl-7-methoxy-4,5-dihydroimidazo[1,2-a]chinazolin-2(1H)-on,

6-Methyl-7-methoxy-4,5-dihydroimidazo[1,2-a]chinazolin-2(1H)-on,

5-Methyl-4,5-dihydroimidazo[1,2-a]chinazolin-2(1H)-on,

6,7-Dichlor-1-methyl-4,5-dihydroimidazo[1,2-a]chinazolin-2(1H)-on,

8-Methyl-4,5-dihydroimidazo[1,2-a]chinazolin-2(1H)-on

und deren Tautomere, sowie Mineralsäuresalze solcher Verbindungen.

Die Erfindung betrifft weiterhin ein Verfahren zur Herstellung der genannten Verbindungen sowie pharmazeutische Präparate auf der Basis der genannten Verbindungen.

Beispiele physiologisch verträglicher Säureadditionssalze sind Mineralsäuresalze, insbesondere Hydrochloride, Hydrobromide, Sulfate und Phosphate.

Die Verbindungen der Formel I können in verschiedenen tautomeren Formen vorliegen. Die Erfindung beschränkt sich daher nicht auf Verbindungen der oben dargestellten Formel I, sondern umfasst auch die Tautomeren, z.B. solche der Formeln

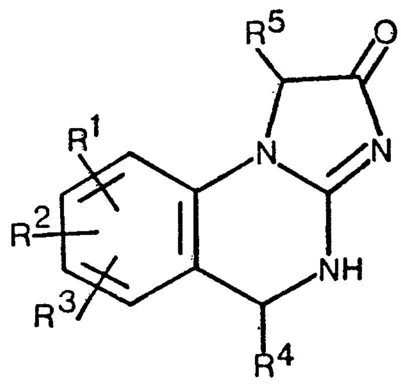

Ia

und

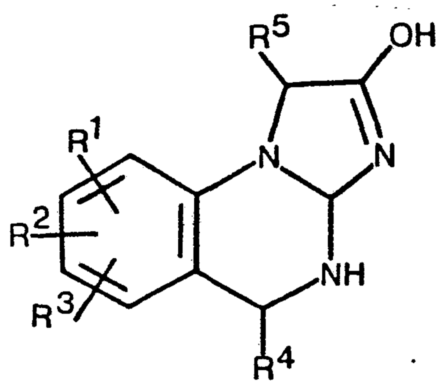

Ib

worin $R^1$-$R^5$ die obigen Bedeutungen haben. Die Verbindungen der Formel I und deren Tautomeren, worin $R^4$ und/oder $R^5$ von Wasserstoff verschieden sind/ist, können weiterhin in Form von Racematen oder in optisch aktiver Form vorliegen, wobei alle diese Formen Gegenstand der Erfindung sind.

Die Verbindungen der Formel I und deren Tautomere, sowie Salze solcher Verbindungen können erfindungsgemäss dadurch hergestellt werden, dass man eine Verbindung der Formel

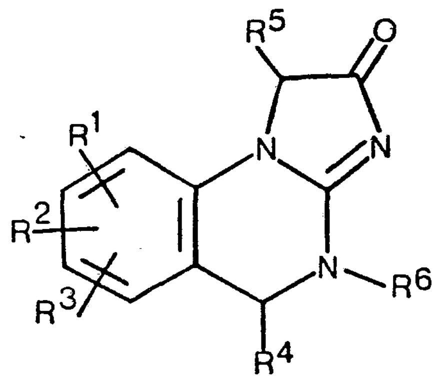

II

worin $R^1$-$R^5$ die in Anspruch 1 angegebenen Bedeutungen haben und $R^6$ eine gegebenenfalls durch $C_{1-4}$-Alkyl oder $C_{1-4}$-Alkoxy ringsubstituierte Benzylgruppe ist, mit einer Säure behandelt und eine erhaltene Verbindung der Formel I oder ein Tautomer davon in dieser Form oder in Form eines physiologisch verträglichen Säureadditionssalzes. isoliert.

Als Säure verwendet man zweckmässigerweise eine Mineralsäure, wie Orthophosphorsäure. Die Umsetzung kann bei einer Temperatur zwischen Raumtemperatur und 150°C, vorzugsweise zwischen 100 und 120°C, vorzugsweise in Anwesenheit von Anisol, durchgeführt werden.

Die Verbindungen der Formel II können durch Umsetzung einer Verbindung der Formel

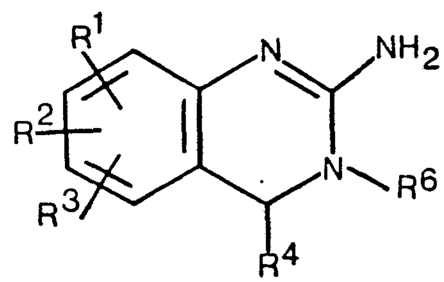

III

mit einem Ester der Formel

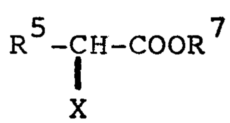

IV

worin $R^1$-$R^6$ die obigen Bedeutungen haben, $R^7$ $C_{1-4}$-Alkyl und X Chlor, Brom oder Jod, vorzugsweise Jod, sind,

zweckmässigerweise in einem organischen Lösungsmittel, wie DMF oder Acetonitril, bei einer Temperatur bis zu Rückflusstemperatur des Reaktionsgemisches, vorzugsweise in Anwesenheit einer anorganischen Base, wie Kaliumcarbonat, hergestellt werden.

Die Verbindungen der Formel III können, in Analogie

zu dem in Chem. Pharm. Bull 28 (1980) 1357-1364 beschriebenen Verfahren zur Herstellung von 3-substituierten 2-Amino-3,4-dihydrochinazolinderivaten ausgehend von 2-Nitrobenzylhalogeniden, nach dem folgenden Reaktionsschema, worin $R^1$-$R^4$ und $R^6$ die obigen Bedeutungen haben, hergestellt werden:

VII

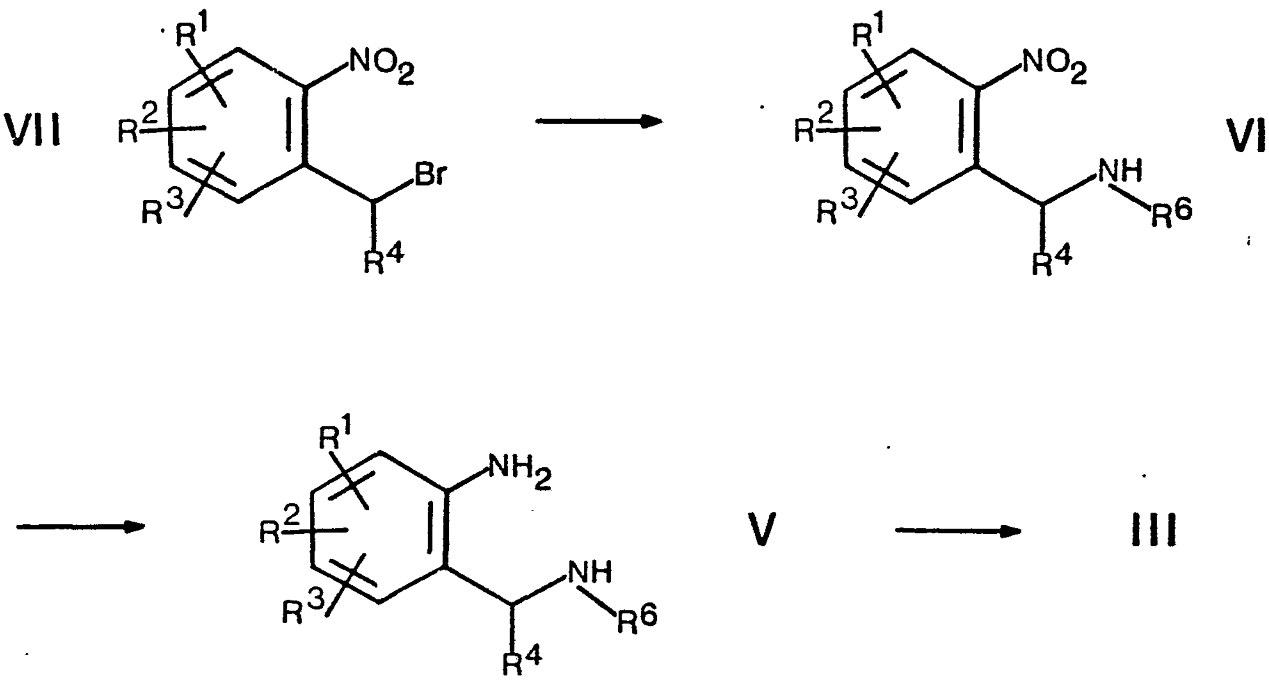

VI

V

III

Die Verbindungen der Formel I, deren Tautomere und physiologisch verträgliche Salze solcher Verbindungen können als Heilmittel Verwendung finden. Sie hemmen z.B. die Aggregation der Blutplättchen und können daher zur Verhütung von Thrombosen verwendet werden. Ferner hemmen sie die Magensäuresekretion und können daher zur Behandlung von Magenulcera Verwendung finden. Ausserdem sind sie kreislaufwirksam. So weisen sie z.B. positiv inotrope Wirkung auf, ohne eine wesentliche Tachycardie zu verursachen.

Die Verbindungen der Formel I und deren Tautomere können als Heilmittel z.B. in Form pharmazeutischer Präparate Verwendung finden, welche sie oder ihre Salze in Mischung mit einem für die enterale, perkutane oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten. Die orale Verabreichung der erfindungsgemässen Verbindungen ist bevorzugt. Für den Erwachsenen kommen eine orale Tagesdosis von 0,5 bis 30 mg/kg und eine parenterale Tagesdosis von 0,05 bis 10 mg/kg in Frage.

Die aggregationshemmende Wirkung wurde nach der Aggregometer-Methode von BORN [Nature 194, 927 (1962)] und MICHAL und BORN [Nature 231, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

Menschliches plättchenreiches Plasma wurde aus citriertem venösem Blut durch Zentrifugieren erhalten. Die Versuche wurden mit Suspensionen der Testsubstanzen in 0,9% NaCl durchgeführt. 0,18 ml Citratplasma wurden mit 10 µl Suspension der Testverbindungen versetzt und 10 Minuten bei 37°C inkubiert, worauf die Aggregation durch Zusatz von 10 µl einer Suspension von Kollagenfibrillen eingeleitet wurde.

Die Resultate sind in der nachstehenden Tabelle wiedergegeben.

Kollagen-induzierte Blutplättchenaggregation

| Verbindung | $EC_{50}$ (µM) |
|---|---|
| 6-Chlor-4,5-dihydroimidazo[1,2-a]chinazolin-2(1H)-on-hydrochlorid | 12,3 |
| 7-Brom-6-methyl-4,5-dihydroimidazo[1,2-a]chinazolin-2(1H)-on-hydrochlorid | 35,0 |

Zur Bestimmung der magensäuresekretionshemmenden Wirkung wurde die nachstehend beschriebene Versuchsanordnung verwendet: weiblichen Ratten, welche 24 Stunden keine Nahrung, jedoch Wasser ad libitum erhalten hatten, wird unter leichter Aethernarkose gemäss Shay et al. [Gastroenterology 5, 43 (1945)] der Pylorus ligiert. Unmittelbar anschliessend wird den Tieren die zu prüfende Substanz intraduodenal verabreicht. Vier Stunden später tötet man die Tiere, bestimmt das Volumen und die Azidität ihres Magensaftes und vergleicht die erhaltenen Werte mit denjenigen von Kontrolltieren, welche gleich behandelt wurden, jedoch keine Testsubstanz erhalten hatten. Als ED 50 bezeichnet man diejenige Dosis der Testsubstanz, die bei den behandelten Tieren im Vergleich zu den Kontrolltieren eine 50%ige Verminderung von Volumen (ED 50-Volumen) bzw. Azidität (ED 50-Azidität) des Magensaftes bewirkt. Für das 6-Chlor-4,5-dihydroimidazo[1,2-a]chinazolin-2(1H)-on-hydrochlorid wurden die folgenden Werte gefunden: ED 50-Volumen = 15 mg/kg i.d. und ED 50-Azidität = 10 mg/kg i.d.

Die positiv inotrope Wirkung wurde nach oraler Gabe der Prüfsubstanzen an wachen Schäferhunden gemessen. Die Tiere sind zu diesem Zweck mit einem implantierten Druck-Telemetrie-System ausgerüstet, wobei der Druckaufnehmer im linken Ventrikel fixiert ist. Der linksventrikuläre Druck wird über den implantierten Radiosender aus dem

Tier gesendet und über ein geeignetes Antennen- und Empfängersystem empfangen, demoduliert und verstärkt. Durch Differenzierung des ansteigenden Schenkels des linksventrikulären Druckes (LVP) wird die maximale Druckanstiegsgeschwindigkeit ($dLVP/dt_{max}$) errechnet, was als Kontraktilitätsparameter gilt. Gleichzeitig wird die Herzfrequenz über einen Cardiotachographen aufgezeichnet. Unter Inotropie werden die prozentuale Veränderung (Δ%) von $dLVP/dt_{max}$ und die Wirkungsdauer in Stunden (Std) angegeben. Unter Tachycardie werden die prozentualen Veränderungen der Herzfrequenz (Δ%) nach Gabe der Prüfsubstanz und die Wirkungsdauer in Stunden (Std) angegeben. Die Resultate sind in der nachstehenden Tabelle wiedergegeben.

**Tabelle**

| Verbindung | Dosis mg/kg | Inotrope Δ% | Inotrope Std. | Herzfrequenz Δ% | Herzfrequenz Std. |
|---|---|---|---|---|---|
| 6-Chlor-4,5-dihydroimidazo[1,2-a]chinazolin-2(1H)-on-hydrochlorid | 10 | +78 | 7 | +77 | 6 |
| 6,7-Dimethyl-4,5-dihydroimidazo[1,2-a]chinazolin-2(1H)-on-hydrochlorid | 10 | +18 | 6 | + 3 | 6 |
| 7-Chlor-4,5-dihydroimidazo[1,2-a]chinazolin-2(1H)-on-hydrochlorid | 10 | +17 | 3,5 | +13 | 3 |
| 7-Methoxy-4,5-dihydroimidazo[1,2-a]chinazolin-2(1H)-on-hydrochlorid | 10 | +67 | 7 | +50 | 6,5 |
| 6,7-Dichlor-4,5-dihydroimidazo[1,2-a]chinazolin-2(1H)-on-hydrochlorid | 1 | +44 | 7,5 | +22 | 7 |
| 6-Methyl-4,5-dihydroimidazo[1,2-a]chinazolin-2(1H)-on-hydrochlorid | 1 | +15 | 2 | - 7 | 3,5 |
| | 3 | +32 | 4,5 | -12 | 5 |
| 9-Methyl-4,5-dihydroimidazo[1,2-a]chinazolin-2(1H)-on-hydrochlorid | 1 | +27 | 2,5 | -19 | 5,5 |
| | 3 | +18 | 6,5 | - 8 | 6 |
| 7-Methyl-4,5-dihydroimidazo[1,2-a]chinazolin-2(1H)-on-hydrochlorid | 3 | +33 | 2 | + 6 | 1 |
| 4,5-Dihydroimidazo[1,2-a]chinazolin-2(1H)-on-hydrochlorid | 3 | +79 | 4 | + 4 | 2,5 |
| 6-Chlor-7-methoxy-4,5-dihydroimidazo[1,2-a]chinazolin-2(1H)-on-hydrochlorid | 1 | +10 | 1 | +47 | 5 |
| | 3 | +32 | 7 | +20 | 6 |

Tabelle (Fortsetzung)

| Verbindung | Dosis mg/kg | Inotrope Δ% | Inotrope Std. | Herzfrequenz Δ% | Herzfrequenz Std. |
|---|---|---|---|---|---|
| | 10 | +60 | 7 | +45 | 7 |
| 7-Chlor-6-methyl-4,5-dihydroimidazo[1,2-a]chinazolin-2(1H)-on-hydrochlorid | 1 | +27 | 6 | + 4 | 5 |
| | 3 | + 7 | 1 | + 6 | 3 |
| | 10 | +50 | 6 | +31 | 6 |
| 7-Brom-6-methyl-4,5-dihydroimidazo[1,2-a]chinazolin-2(1H)-on-hydrochlorid | 1 | + 8 | 2,5 | +10 | 4 |
| | 3 | +16 | 5,5 | 0 | 4 |
| | 10 | +120 | 8 | +59 | 8 |
| 6,7-Dichlor-1-methyl-4,5-dihydro[1,2-a]chinazolin-2(1H)-on-hydrochlorid | 3 | +48 | 8 | +16 | 8 |

Beispiel 1

Eine Lösung von 0,1 mol 6-Chlor-4-(p-methoxybenzyl)-5H-imidazo[1,2-a]chinazolin-2(1H)-on in 1,2 l 85%iger Phosphorsäure und 60 ml Anisol wird unter Rühren 5 Stunden auf 120° erhitzt. Dann wird abgekühlt, auf 6 l Eiswasser gegossen und mit konz. Ammoniaklösung alkalisch gestellt. Der Niederschlag von 6-Chlor-4,5-dihydroimidazo[1,2-a]chinazolin-2(1H)-on wird abfiltriert und mit Wasser gewaschen. Ausbeute: 60-80%.

Zur Herstellung des Hydrochlorides wird die freie Base in Aethanol unter Zugabe einer äquivalenten Menge 25%-iger Salzsäure in der Wärme gelöst und durch langsames Abkühlen auskristallisieren gelassen, Smp. 305° (Zersetzung).

Das Ausgangsmaterial kann wie folgt hergestellt werden:

Eine Lösung von 100 g 2-Chlor-6-nitrobenzylbromid in 600 ml Methanol wird bei Raumtemperatur zu einer Lösung von 200 g p-Methoxybenzylamin in 600 ml Methanol getropft. Nach 5 Stunden wird der grösste Teil des Methanols abgedampft und der Rückstand auf Eiswasser gegossen. Der Niederschlag von 2-Chlor-4'-methoxy-6-nitrodibenzylamin wird abfiltriert. Durch Lösen in Methanol-Salzsäure wird das Hydrochlorid hergestellt, das aus Methanol/Aethylacetat umkristallisiert wird, Smp. 183-186°.

Eine Lösung von 100 g 2-Chlor-4'-methoxy-6-nitrodibenzylamin-hydrochlorid wird unter Zusatz von 50 ml Triäthylamin in 1 l Aethanol in Anwesenheit von Raney-Nickel hydriert. Dann wird vom Katalysator abfiltriert, das Filtrat eingedampft, in 300 ml wässrigem Ammoniak suspendiert und mit Aether extrahiert. Die Aetherlösung wird abgedampft und das zurückbleibende 2-Amino-6-chlor-4'-methoxydibenzylamin durch Lösen in Methanol und behandeln mit HCl in das Monohydrochlorid, Smp. 127-129° übergeführt.

55 g 2-Amino-6-chlor-4'-methoxydibenzylamin werden in 1 l Dioxan gelöst und mit 21,5 g Bromcyan 4 Tage bei Raumtemperatur gerührt. Dann wird mit 1 l Aether verdünnt und 47,5 g von 2-Amino-5-chlor-3,4-dihydro-3-(p-methoxybenzyl)-chinazolin-hydrobromid, Smp. 245-247°, abfiltriert.

Eine Lösung von 0,15 mol 2-Amino-5-chlor-3,4-dihydro-3-(p-methoxybenzyl)-chinazolin-hydrobromid in 800 ml DMF wird mit 0,18 mol Jodessigsäureäthylester und 0,2 mol Kaliumcarbonat versetzt und 18 Stunden bei Raumtemperatur gerührt. Dann wird noch 5 Stunden bei 90° gerührt, abgekühlt und auf 5 l Eiswasser gegossen. Das ausgefallene Produkt, 6-Chlor-4-(p-methoxybenzyl)-5H-imidazo[1,2-a]-chinazolin-2(1H)-on, wird abfiltriert und gut mit Wasser gewaschen. Zur Herstellung des Hydrochlorides wird aus Aethanol-Salzsäure umkristallisiert, Smp. 230° (Zers.).

## Beispiel 2

In zu Beispiel 1 analoger Weise wird

aus 6,7-Dimethyl-4-(p-methoxybenzyl)-5H-imidazo[1,2-a]chinazolin-2(1H)-on, Smp. des Hydrochlorids 225-226° (Zers.),
das 6,7-Dimethyl-4,5-dihydroimidazo[1,2-a]chinazolin-2(1H)-on-hydrochlorid, Smp. < 300° (Zers.);
aus 7-Chlor-4-(p-methoxybenzyl)-5H-imidazo[1,2-a]chinazolin-2(1H)-on, Smp. des Hydrochlorids 228-229° (Zers.),
das 7-Chlor-4,5-dihydroimidazo[1,2-a]chinazolin-2(1H)-on-hydrochlorid, Smp. 295-297° (Zers.);
aus 7-Methoxy-4-(p-methoxybenzyl)-5H-imidazo[1,2-a]chinazolin-2(1H)-on, Smp. des Hydrochlorids 244-245° (Zers.),
das 7-Methoxy-4,5-dihydroimidazo[1,2-a]chinazolin-2(1H)-on-hydrochlorid, Smp. 290° (Zers.);
aus 6,7-Dichlor-4-(p-methoxybenzyl)-5H-imidazo[1,2-a]chinazolin-2(1H)-on, Smp. des Hydrochlorids 237-238° (Zers.),
das 6,7-Dichlor-4,5-dihydroimidazo[1,2-a]chinazolin-2(1H)-on-hydrochlorid, Smp. < 300° (Zers.);

aus 6-Methyl-4-(p-methoxybenzyl)-5H-imidazo[1,2-a]chinazolin-2(1H)-on, Smp. des Hydrochlorids 240-245° (Zers.),

das 6-Methyl-4,5-dihydroimidazo[1,2-a]chinazolin-2(1H)-on-hydrochlorid, Smp. <300° (Zers.);

aus 9-Methyl-4-(p-methoxybenzyl)-5H-imidazo[1,2-a]chinazolin-2(1H)-on, Smp. des Hydrochlorids 228-229° (Zers.),

das 9-Methyl-4,5-dihydroimidazo[1,2-a]chinazolin-2(1H)-on-hydrochlorid, Smp. 270-272° (Zers.);

aus 8-Chlor-4-(p-methoxybenzyl)-5H-imidazo[1,2-a]chinazolin-2(1H)-on, Smp. des Hydrochlorids 231-234° (Zers.),

das 8-Chlor-4,5-dihydroimidazo[1,2-a]chinazolin-2(1H)-on-hydrochlorid, Smp. < 300° (Zers.);

aus 6,7,8-Trimethoxy-4-(p-methoxybenzyl)-5H-imidazo[1,2-a]chinazolin-2(1H)-on, Smp. des Hydrochlorids 230-231° Zers.),

das 6,7,8-Trimethoxy-4,5-dihydroimidazo[1,2-a]chinazolin-2(1H)-on-hydrochlorid, Smp. 250° (Zers.);

aus 6,7-Dimethoxy-4-(p-methoxybenzyl)-5H-imidazo[1,2-a]chinazolin-2(1H)-on, Smp. des Hydrochlorids 255-256° (Zers.),

das 6,7-Dimethoxy-4,5-dihydroimidazo[1,2-a]chinazolin-2(1H)-on-hydrochlorid, Smp. 258° (Zers.);

aus 7-Methyl-4-(p-methoxybenzyl)-5H-imidazo[1,2-a]chinazolin-2(1H)-on, Smp. des Hydrochlorids 236-237° (Zers.),

das 7-Methyl-4,5-dihydroimidazo[1,2-a]chinazolin-2(1H)-on-hydrochlorid, Smp. < 300° (Zers.);

aus 4-(p-Methoxybenzyl)-5H-imidazo[1,2-a]chinazolin-2(1H)-on, Smp. des Hydrochlorids 226-227° (Zers.),

das 4,5-Dihydroimidazo[1,2-a]chinazolin-2(1H)-on-hydrochlorid, Smp. <300° (Zers.);

aus 6-Chlor-7-methoxy-4-(p-methoxybenzyl)-5H-imidazo[1,2-a]chinazolin-2(1H)-on, Smp. des Hydrochlorids 262° (Zers.),

das 6-Chlor-7-methoxy-4,5-dihydroimidazo[1,2-a]chinazolin-2(1H)-on-hydrochlorid, Smp. 290° (Zers.);

aus 7-Chlor-6-methyl-4-(p-methoxybenzyl)-5H-imidazo[1,2-a]chinazolin-2(1H)-on, Smp. des Hydrochlorids 231-232° (Zers.),

das 7-Chlor-6-methyl-4,5-dihydroimidazo[1,2-a]chinazolin-2(1H)-on-hydrochlorid, Smp. ∠ 300° (Zers.);

aus 7-Brom-6-methyl-4-(p-methoxybenzyl)-5H-imidazo [1,2-a]chinazolin-2(1H)-on, Smp. des Hydrochlorids 252-254° (Zers.),

das 7-Brom-6-methyl-4,5-dihydroimidazo[1,2-a]chinazolin-2(1H)-on-hydrochlorid, Smp. ∠ 300° (Zers.);

aus 1-Methyl-6-chlor-7-methoxy-4-(p-methoxybenzyl)-5H-imidazo[1,2-a]chinazolin-2(1H)-on, Smp. 252° (Zers.),

das 1-Methyl-6-chlor-7-methoxy-4,5-dihydroimidazo [1,2-a]chinazolin-2(1H)-on-hydrochlorid, Smp. 250°C (Zers.);

aus 6,7-Dichlor-1-methyl-4-(p-methoxybenzyl)-5H-imidazo [1,2-a]chinazolin-2(1H)-on, Smp. des Hydrochlorids 212-214°,

das 6,7-Dichlor-1-methyl-4,5-dihydroimidazo[1,2-a]chinazolin-2(1H)-on-hydrochlorid, Smp. 284-289°;

aus 5-Methyl-4-(p-methoxybenzyl)-5H-imidazo[1,2-a]chinazolin-2(1H)-on, Smp. des Hydrochlorids 227-228°,

das 5-Methyl-4,5-dihydroimidazo[1,2-a]chinazolin-2(1H)-on-hydrochlorid, Smp. 280° (Zers.) hergestellt.

## Beispiel 3

In zu Beispiel 1 analoger Weise werden folgende Verbindungen hergestellt:

5-Methyl-7-methoxy-4,5-dihydroimidazo[1,2-a]chinazolin-2(1H)-on,

6-Methyl-7-methoxy-4,5-dihydroimidazo[1,2-a]chinazolin-2(1H)-on, Smp. des Hydrochlorids 285-287° (Zers.),

8-Methyl-4,5-dihydroimidazo[1,2-a]chinazolin-2(1H)-on, Smp. des Hydrochlorids 300° (Zers.).

## Beispiel 4

In üblicher Weise werden Tabletten folgender Zusammensetzung hergestellt:

| | |
|---|---|
| 6-Chlor-4,5-dihydroimidazo[1,2-a]chinazolin-2(1H)-on-hydrochlorid | 185,0 mg |
| Milchzucker | 15,0 mg |
| Maisstärke | 37,5 mg |
| Wasserlösliches Polyvinylpyrrolidon | 10,0 mg |
| Magnesiumstearat | 2,5 mg |
| Gesamtgewicht pro Tablette | 250,0 mg |

## Beispiel 5

In üblicher Weise werden Gelatinesteckkapseln folgender Zusammensetzung hergestellt:

| | |
|---|---|
| 6-Chlor-4,5-dihydroimidazo[1,2-a]chinazolin-2(1H)-on-hydrochlorid | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

## Beispiel 6

In üblicher Weise wird eine Injektionslösung folgender Zusammensetzung hergestellt:

| | |
|---|---|
| 6-Chlor-4,5-dihydroimidazo[1,2-a]chinazolin-2(1H)-on-hydrochlorid | 115,0 mg |
| Glycerinformal | 2,4 ml |
| Wasser | 4,0 ml |

## Patentansprüche

1. Imidazochinazoline der Formel

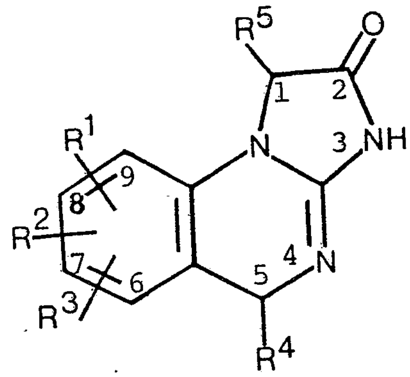

I

worin $R^1$, $R^2$ und $R^3$ Wasserstoff, Halogen, $C_{1-4}$-Alkyl, $C_{1-4}$-Alkoxy oder $C_{2-5}$-Alkoxyalkyl, oder zwei benachbarte Reste $R^1$, $R^2$ oder $R^3$ Methylendioxy oder Aethylendioxy, und $R^4$ und $R^5$ Wasserstoff oder $C_{1-4}$-Alkyl sind, und deren Tautomere, sowie physiologisch verträgliche Säureadditionssalze solcher Verbindungen.

2. Verbindungen nach Anspruch 1, worin $R^1$ und $R^2$ Wasserstoff, Halogen, $C_{1-4}$-Alkyl oder $C_{1-4}$-Alkoxy in 6- und 7-Stellung und $R^3$ Wasserstoff sind.

3. Verbindungen nach Anspruch 2, worin $R^1$ und $R^2$ Wasserstoff, Chlor, Brom, Methyl oder Methoxy in 6- und 7-Stellung und $R^3$ Wasserstoff sind.

4. Verbindungen nach Anspruch 3, worin $R^1$ 6-Chlor, $R^2$ 7-Chlor und $R^3$ Wasserstoff ist.

5. Verbindungen nach einem der Ansprüche 1-4, worin $R^4$ und $R^5$ Wasserstoff oder Methyl sind.

6. 6,7-Dichlor-4,5-dihydroimidazo[1,2-a]chinazolin-2(1H)-on und deren Tautomere, sowie physiologisch verträgliche Säureadditionssalze solcher Verbindungen.

7. 6-Chlor-4,5-dihydroimidazo[1,2-a]chinazolin-2(1H)-on und deren Tautomere, sowie physiologisch verträgliche Säureadditionssalze solcher Verbindungen.

8. Eine Verbindung aus der Gruppe der folgenden:

7-Brom-6-methyl-4,5-dihydroimidazo[1,2-a]chinazolin-2(1H)-on,
6,7-Dimethyl-4,5-dihydroimidazo[1,2-a]chinazolin-2(1H)-on,
7-Chlor-4,5-dihydroimidazo[1,2-a]chinazolin-2(1H)-on,
7-Methoxy-4,5-dihydroimidazo[1,2-a]chinazolin-2(1H)-on,
7-Chlor-6-methyl-4,5-dihydroimidazo[1,2-a]chinazolin-2(1H)-on,
7-Methyl-4,5-dihydroimidazo[1,2-a]chinazolin-2(1H)-on,
6-Methyl-4,5-dihydroimidazo[1,2-a]chinazolin-2(1H)-on,
4,5-Dihydroimidazo[1,2-a]chinazolin-2(1H)-on,
6,7,8-Trimethoxy-4,5-dihydroimidazo[1,2-a]chinazolin-2(1H)-on,
6-Chlor-7-methoxy-4,5-dihydroimidazo[1,2-a]chinazolin-2(1H)-on,
8-Chlor-4,5-dihydroimidazo[1,2-a]chinazolin-2(1H)-on,
9-Methyl-4,5-dihydroimidazo[1,2-a]chinazolin-2(1H)-on,
6,7-Dimethoxy-4,5-dihydroimidazo[1,2-a]chinazolin-2(1H)-on,
1-Methyl-6-chlor-7-methoxy-4,5-dihydroimidazo[1,2-a]chinazolin-2(1H)-on,
5-Methyl-7-methoxy-4,5-dihydroimidazo[1,2-a]chinazolin-2(1H)-on,
6-Methyl-7-methoxy-4,5-dihydroimidazo[1,2-a]chinazolin-2(1H)-on,
5-Methyl-4,5-dihydroimidazo[1,2-a]chinazolin-2(1H)-on,
6,7-Dichlor-1-methyl-4,5-dihydroimidazo[1,2-a]chinazolin-2(1H)-on,
8-Methyl-4,5-dihydroimidazo[1,2-a]chinazolin-2(1H)-on
und deren Tautomere, sowie physiologisch verträgliche Säureadditionssalze solcher Verbindungen.

9. Eine Verbindung nach einem der Ansprüche 1-8 als pharmazeutischer Wirkstoff.

10. Eine Verbindung nach einem der Ansprüche 1-8 als die Blutplättchenaggregation hemmender Wirkstoff.

11. Eine Verbindung nach einem der Ansprüche 1-8 als die Magensäuresekretion hemmender Wirkstoff.

12. Eine Verbindung nach einem der Ansprüche 1-8 als kreislaufwirksames Mittel.

13. Pharmazeutisches Präparat enthaltend eine Verbindung nach einem der Ansprüche 1-8.

14. Pharmazeutisches Präparat zur Hemmung der Blutplättchenaggregation, enthaltend eine Verbindung nach einem der Ansprüche 1-8.

15. Pharmazeutisches Präparat zur Hemmung der Magensäuresekretion, enthaltend eine Verbindung nach einem der Ansprüche 1-8.

16. Kreislaufwirksames Präparat enthaltend eine Verbindung nach einem der Ansprüche 1-8.

17. Verfahren zur Herstellung von Imidazochinazolinen der Formel I in Anspruch 1 und von deren Tautomeren, sowie von physiologisch verträglichen Säureadditionssalzen solcher Verbindungen, dadurch gekennzeichnet, dass man eine Verbindung der Formel

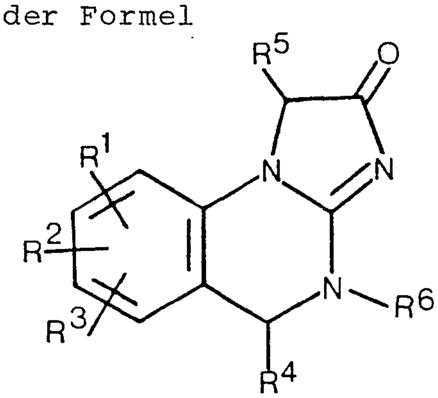

II

worin $R^1$-$R^5$ die in Anspruch 1 angegebenen Bedeutungen haben und $R^6$ eine gegebenenfalls durch $C_{1-4}$-Alkyl oder $C_{1-4}$-Alkoxy ringsubstituierte Benzylgruppe ist,

mit einer Säure umsetzt und eine erhaltene Verbindung der Formel I oder ein Tautomer davon in dieser Form oder in Form eines physiologisch verträglichen Säureadditionssalzes isoliert.

***

Patentansprüche für Oesterreich

1. Verfahren zur Herstellung von neuen Imidazochinazolinen der Formel

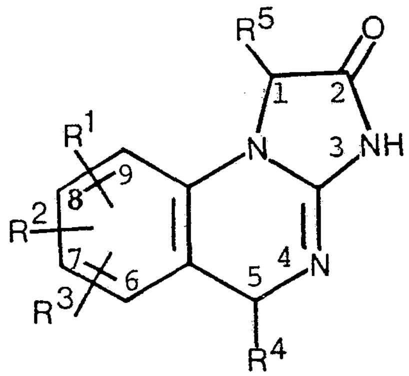

I

worin $R^1$, $R^2$ und $R^3$ Wasserstoff, Halogen, $C_{1-4}$-Alkyl, $C_{1-4}$-Alkoxy oder $C_{2-5}$-Alkoxyalkyl, oder zwei benachbarte Reste $R^1$, $R^2$ oder $R^3$ Methylendioxy oder Aethylendioxy, und $R^4$ und $R^5$ Wasserstoff oder $C_{1-4}$-Alkyl sind, und deren Tautomere, sowie physiologisch verträgliche Säureadditionssalze solcher Verbindungen, dadurch gekennzeichnet, dass man eine Verbindung der Formel

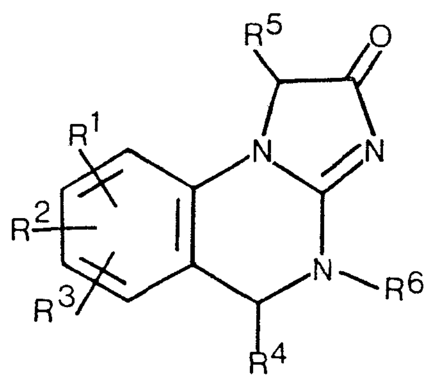

II

worin $R^1$-$R^5$ die oben angegebenen Bedeutungen haben und $R^6$ eine gegebenenfalls durch $C_{1-4}$-Alkyl oder $C_{1-4}$-Alkoxy ringsubstituierte Benzylgruppe ist, mit einer Säure umsetzt und eine erhaltene Verbindung der Formel I oder ein Tautomer davon in dieser Form oder in Form eines physiologisch verträglichen Säureadditionssalzes isoliert.

2. Verfahren nach Anspruch 1, dadurch gekennzeichnet, dass man von einer Verbindung der Formel II ausgeht, worin $R^1$ und $R^2$ Wasserstoff, Halogen, $C_{1-4}$-Alkyl oder $C_{1-4}$-Alkoxy in 6- und 7-Stellung und $R^3$ Wasserstoff sind.

3. Verfahren nach Anspruch 2, dadurch gekennzeichnet, dass man von einer Verbindung der Formel II ausgeht, worin $R^1$ und $R^2$ Wasserstoff, Chlor, Brom, Methyl oder Methoxy in 6- und 7-Stellung und $R^3$ Wasserstoff sind.

4. Verfahren nach Anspruch 3, dadurch gekennzeichnet, dass man von einer Verbindung der Formel II ausgeht, worin $R^1$ 6-Chlor , $R^2$ 7-Chlor und $R^3$ Wasserstoff ist.

5. Verfahren nach einem der Ansprüche 1-4, dadurch gekennzeichnet, dass man von einer Verbindung der Formel II ausgeht, worin $R^4$ und $R^5$ Wasserstoff oder Methyl sind.

6. Verfahren nach einem der Ansprüche 1-5, dadurch gekennzeichnet, dass man als Ausgangsmaterial das 6,7-Dichlor-4-(p-methoxybenzyl)-5H-imidazo[1,2-a]chinazolin-2(1H)-on verwendet.

7. Verfahren nach einem der Ansprüche 1-3 oder 5, dadurch gekennzeichnet, dass man als Ausgangsmaterial das 6-Chlor-4-(p-methoxybenzyl)-5H-imidazo[1,2-a]chinazolin-2(1H)-on verwendet.

8. Verfahren nach Anspruch 1, dadurch gekennzeichnet, dass man

7-Brom-6-methyl-4,5-dihydroimidazo[1,2-a]chinazolin-2(1H)-on,

6,7-Dimethyl-4,5-dihydroimidazo[1,2-a]chinazolin-2(1H)-on,

7-Chlor-4,5-dihydroimidazo[1,2-a]chinazolin-2(1H)-on,

7-Methoxy-4,5-dihydroimidazo[1,2-a]chinazolin-2(1H)-on,
7-Chlor-6-methyl-4,5-dihydroimidazo[1,2-a]chinazolin-2(1H)-on,
7-Methyl-4,5-dihydroimidazo[1,2-a]chinazolin-2(1H)-on,
6-Methyl-4,5-dihydroimidazo[1,2-a]chinazolin-2(1H)-on,
4,5-Dihydroimidazo[1,2-a]chinazolin-2(1H)-on,
6,7,8-Trimethoxy-4,5-dihydroimidazo[1,2-a]chinazolin-2(1H)-on,
6-Chlor-7-methoxy-4,5-dihydroimidazo[1,2-a]chinazolin-2(1H)-on,
8-Chlor-4,5-dihydroimidazo[1,2-a]chinazolin-2(1H)-on,
9-Methyl-4,5-dihydroimidazo[1,2-a]chinazolin-2(1H)-on,
6,7-Dimethoxy-4,5-dihydroimidazo[1,2-a]chinazolin-2(1H)-on,
1-Methyl-6-chlor-7-methoxy-4,5-dihydroimidazo[1,2-a]chinazolin-2(1H)-on,
5-Methyl-7-methoxy-4,5-dihydroimidazo[1,2-a]chinazolin-2(1H)-on,
6-Methyl-7-methoxy-4,5-dihydroimidazo[1,2-a]chinazolin-2(1H)-on,
5-Methyl-4,5-dihydroimidazo[1,2-a]chinazolin-2(1H)-on,
6,7-Dichlor-1-methyl-4,5-dihydroimidazo[1,2-a]chinazolin-2(1H)-on,
8-Methyl-4,5-dihydroimidazo[1,2-a]chinazolin-2(1H)-on
und deren Tautomere, sowie physiologisch verträgliche Säureadditionssalze solcher Verbindungen ausgehend von der entsprechenden Verbindung der Formel II herstellt.

Europäisches Patentamt

**EUROPÄISCHER RECHERCHENBERICHT**

0046267

Nummer der Anmeldung

EP 81 10 6257

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | DE - A - 2 305 575 (BRISTOL-MYERS)<br>---- | 1,9 | C 07 D 487/04<br>491/14<br>A 61 K 31/505//<br>C 07 C 87/28<br>C 07 D 239/84<br>(C 07 D 487/04<br>239/00<br>235/00) |
| | | | RECHERCHIERTE SACHGEBIETE (Int. Cl.3) |
| | | | C 07 D 487/04<br>A 61 K 31/505 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 19-10-1981 | ALFARO |